# EUROPEAN PATENT APPLICATION

(11) **EP 2 258 426 A1**
(43) Date of publication of application: **08.12.2010**
(21) Application number: 09724924.7
(22) Date of filing: 23.03.2009
(51) Int. Cl.: A61M 5/28

(54) **DRUG CONTAINER**

(30) Priority: 28.03.2008 JP 2008088057
(71) Applicant: Terumo Kabushiki Kaisha, Shibuya-ku Tokyo 151-0072 (JP)
(72) Inventor: MATSUMOTO, Atsushi, Ashigarakami-gun Kanagawa 259-0151 (JP)
(74) Representative: TBK-Patent
(86) International application number: PCT/JP2009/055649
(87) International publication number: WO 2009/119496

(57) **Abstract**

In a drug container filled with a liquid drug, bubbles are formed during prolonged storage even in the case where the liquid drug is filled in a gas-free state after removing dissolved gas therefrom. To solve the problem, bubbles formed in the container during storage are removed and the formation of bubbles in the container is prevented. A drug container, in which a liquid drug is enclosed, provided at least in a part thereof with a resin member coming into contact with the liquid drug as described above, **characterized in that** a pressurizing member removable from the drug container, by which the liquid drug as described above is maintained in a pressurized state, is provided and the liquid drug as described above is filled in a substantially bubble-free state.

## Description

### Technical Field

The present invention relates to a container filled with a liquid drug, for use in storage and transportation of the liquid drug. Particularly, the invention relates to a container and a prefilled syringe which eliminate the need to transfer a liquid drug into another injection implement at the time of use.

### Background Art

In recent years, in view of such problems as contamination at the times of dissolution or transfer between containers, prefilled syringes having both a function as a container and a function as an injection implement have come to be used. A prefilled syringe, unlike a vial container, does not need transfer of a drug into an injection implement such as a syringe and a pump. Thus, the prefilled syringe is advantageous in that it can be used for injection immediately upon unsealing thereof.

Besides, in relation to protein preparations such as insulin, containers which can, when set on an exclusive-use pump or injection implement with a gasket disposed at one end of the vial, be utilized for injection while preventing a solution in the inside thereof from making contact with the outside air, have been developed.

### Disclosure of Invention

### Problem to be Solved by the Invention

In the case of a container utilized directly as an injection implement or a part thereof, as above-mentioned, there may arise the problem of generation of bubbles in the container during storage.

Normally, in the case where a liquid-containing container is made of a gas-permeable material, for example, a resin such as polypropylene frequently used for forming drug containers, bubbles are generated during prolonged storage even if a liquid drug is filled and stored in a gas-free state after removing dissolved gas therefrom.

This arises from the fact that even if a liquid drug can be degassed to a substantially gas-free state when the liquid-containing container is filled with the liquid drug, external gas permeates through the resin constituting the container, and the gas brought to supersaturation due to a temperature variation during storage is evolved as bubbles inside the container.

This phenomenon occurs to some degree in use of water as a solvent, since such gases as oxygen and nitrogen are dissolved in water. Therefore, the phenomenon can occur in drugs using water as a solvent.

The bubbles are discharged before using a prefilled syringe, which is used for injection or pump injection immediately upon unsealing, so that operations for using such a prefilled syringe are intricate.

Besides, in the cases of drugs to be injected in extremely small amounts such as an insulin preparation, mixing-in of bubbles may lead to an insufficient dose or compression of bubbles under pressure may lead to an inaccurate ejection amount.

In addition, where a water-soluble protein preparation is stored and transported in a solution state, a condition where the container is not filled up with the liquid, that is, a condition where a gas region or bubbles are present inside the container produces the following problem. The aqueous solution is stirred due for example to vibrations during transportation, and the surface tension of bubbles exerts a load on the molecular structure of the protein, whereby denaturation (degeneration) of the protein is promoted and the preservability is worsened.

For preventing such denaturation, in the case of an insulin container to be used in the state of being set on the above-mentioned exclusive-use pump or injection implement, the insulin should be placed in a gas-impermeable glass container in a gas-free state and should be stored at a low temperature and in a condition where the temperature is little varied.

Since such a liquid drug container is used in the state of direct connection with a pump or injection implement, however, a resin seal (gasket) slidable while keeping a liquid-tight sealed state between itself and the liquid drug container is needed for pushing the liquid drug out of the container. In addition, other components such as a resin septum to be penetrated by a connection end part of an injection needle or a connection needle constituting a route of ejection of the liquid drug from the liquid drug container are needed. Accordingly, it has been impossible to perfectly prevent the generation of bubbles which might arise from penetration of the outside air into a liquid drug container.

### Means for Solving the Problem

It is an object of the present invention to provide a system in which a liquid drug is stored in a pressurized state, thereby removing gaseous components through a resin constituting a wall part of a container, and ensuring that the drug container filled with the liquid drug can be used in a bubble-free condition.

The present inventor found out that, by putting a liquid drug in a predetermined pressurized state, it is possible to prevent the problem that a gas having entered into a container by permeating through a resin constituting a wall part of the container might be supersaturated under temperature variations during storage, to form bubbles in the container. Or, even if bubbles should be formed in the container during storage, it would be possible by this method to cause the bubbles to disappear. Based on this finding, the present invention has been completed.

The above object is attained by the invention as described in the following (1) to (6).

(1) A drug container, in which a liquid drug is enclosed, provided at least in a part thereof with a resin coming into contact with the liquid drug, wherein a pressurizing member removable from the drug container and operable to maintain the liquid drug in a pressurized state is provided, and the liquid drug is filled in a substantially bubble-free state.

Thus, the drug container having the resin member is provided with the removable pressurizing member. This makes it possible to remove the bubbles present in the drug container to the exterior of the container and to dissolve the bubbles into the liquid drug.

(2) The drug container according to (1) above, wherein the drug container includes an outer tube having a tip and a rear end, and a seal slidable on an inner surface of the outer tube in an axial direction of the outer tube, the tip being sealed with a sealing member, at least one of the seal and the outer tube includes the resin member, and the liquid drug is placed in the outer tube between the sealing member and the seal.

Thus, in the drug container having the resin member, the liquid drug is filled between the sealing member and the seal. This permits the drug container according to the present invention to be used as a prefilled syringe.

(3) The drug container according to (2) above, wherein a plunger extending from the rear end side of the outer tube and having a tip part and a rear end part is provided, the tip part being connected with the seal, the rear end part having a pressing part; and the pressuring member is an elastic member which is removably mounted by a flange's surface on the side of the tip of the outer tube and the pressing part and which gives a compressive force between the flange and the pressing part.

Thus, the flange of the syringe and the plunger are clamped and a compressive force is given therebetween. This ensures that the drug container capable of producing the effect of the present invention can be fabricated as a prefilled syringe with a simple configuration.

(4) The drug container according to (2) above, wherein the pressurizing member includes an elastic member which has a fixation part for fixation to the outer tube and which is detachably attached to the seal.

Thus, the pressurizing member is provided with the fixation part and the elastic member detachably attached to the seal. This permits the drug container according to the present invention to be used as an insulin injection device, such as an insulin pen.

(5) The drug container according to (2) above, wherein the pressurizing member has a fixation part for fixation to the outer tube, and maintains the liquid drug in a pressurized state through pressing of an elastic member of the seal provided with the elastic member.

Thus, the pressurizing member presses the elastic member provided in the seal. This permits the drug container to be used as an insulin injection device, such as an insulin pen.

(6) The drug container according to any of (1) to (5) above, wherein an internal pressure relevant to the pressurized state in which the liquid drug is maintained is 10 to 90 kPa.

With such a configuration, the condition where bubbles are absent in the drug container can be attained more securely.

### Effect of the Invention

According to the present invention, a liquid drug can be stored while maintaining the liquid drug in a pressurized state until immediately before the use thereof, and penetration of gas through a resin member constituting the container can be prevented from occurring during storage. Consequently, it is possible to provide a drug container filled with a liquid drug which is substantially bubble-free.

In addition, the liquid drug is stored in a pressurized state by a pressurizing member by which a seal (gasket) is held in a pressurized state, and bubbles in the drug container are removed to the exterior of the container and dissolved into the liquid drug, whereby a drug container with no bubble present therein can be provided.

### Brief Description of Drawings

[FIG. 1]

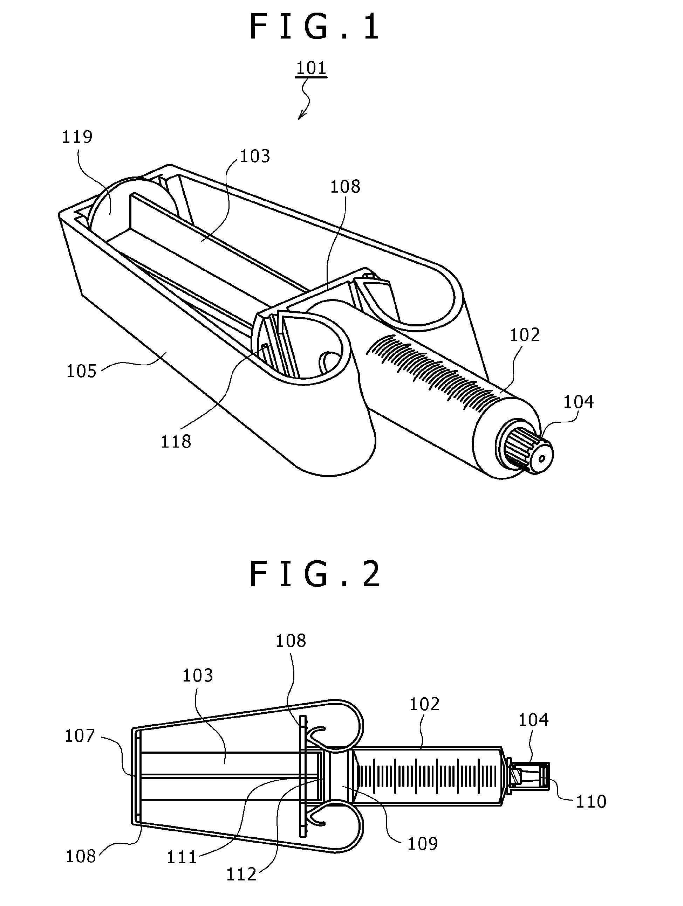

   FIG. 1 is a perspective view of a prefilled syringe according to an embodiment of the present invention.
[FIG. 2]
   FIG. 2 is a plan view of the prefilled syringe shown in FIG. 1. (The vicinity of the tip of the syringe is shown in a cut-away sectional view.)
[FIG. 3]

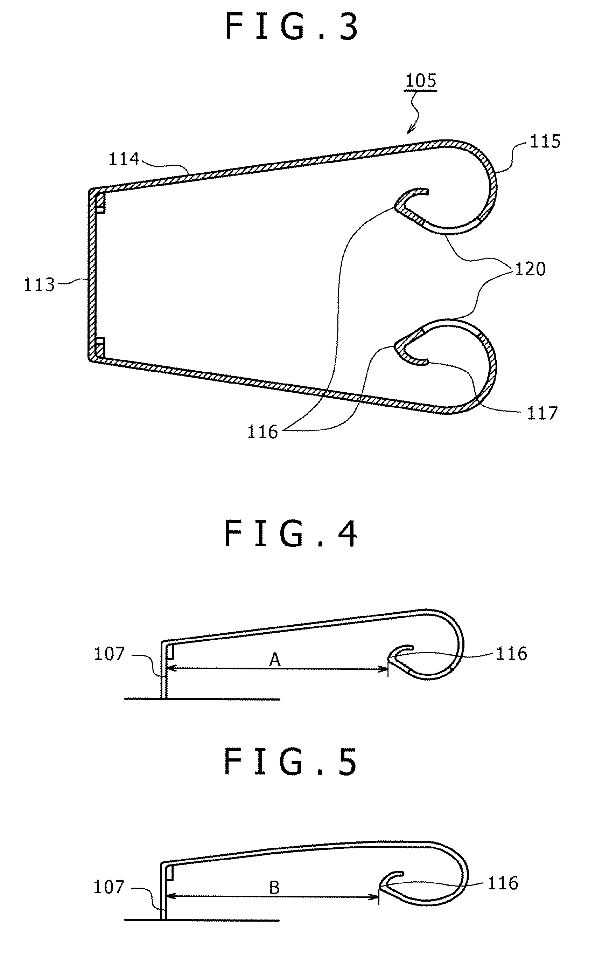

   FIG. 3 is a sectional view of a spring member used in the prefilled syringe of FIG. 1.
[FIG. 4]
   FIG. 4 is a partial view of the spring member of the prefilled syringe of FIG. 1, in a mounted state.
[FIG. 5]
   FIG. 5 is a partial view of the spring member of the prefilled syringe of FIG. 1, in a dismounted state.
[FIG. 6]

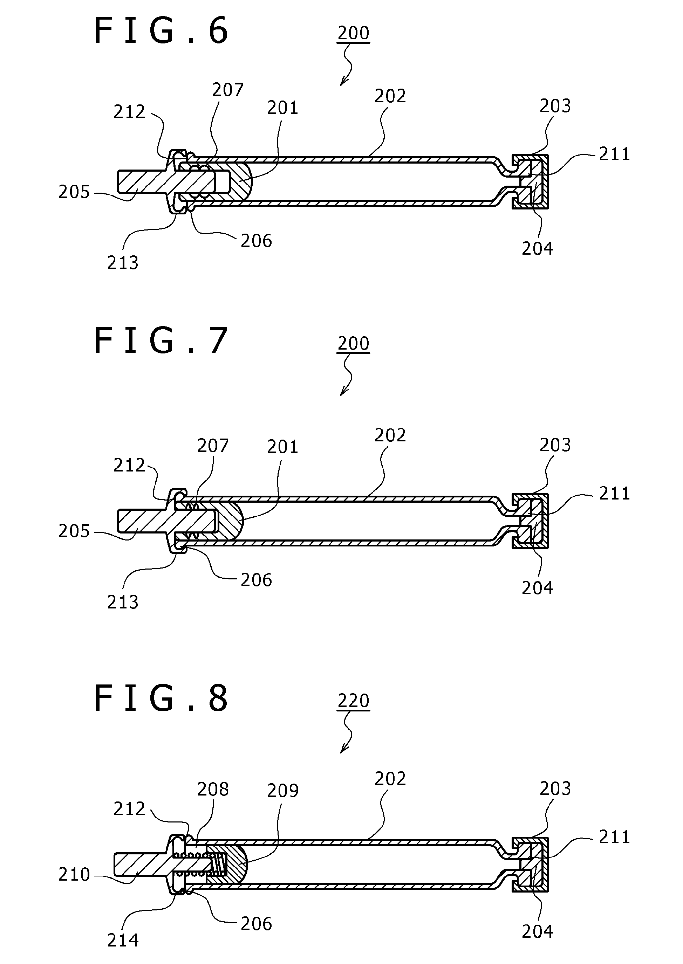

   FIG. 6 is a sectional view of a container for connection to a device, before fitting of a fixing cap according to a second embodiment of the present invention.
[FIG. 7]
   FIG. 7 is a sectional view of the container for connection to a device, after fitting of the fixing cap according to the embodiment shown in FIG. 6.
[FIG. 8]
   FIG. 8 is a sectional view of the container for connection to a device, before fitting of a fixing cap according to a modification of the second embodiment of the present invention.
[FIG. 9]

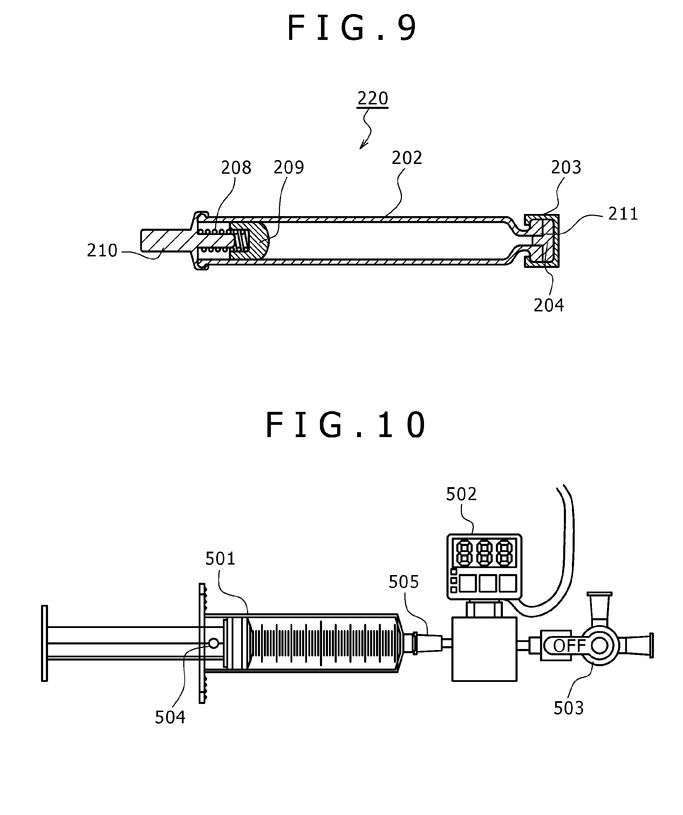

   FIG. 9 is a sectional view of the container for connection to a device, after fitting of the fixing cap according to the embodiment shown in FIG. 8.
[FIG. 10]
   FIG. 10 illustrates a measuring apparatus used in Experimental Examples.
[FIG. 11]

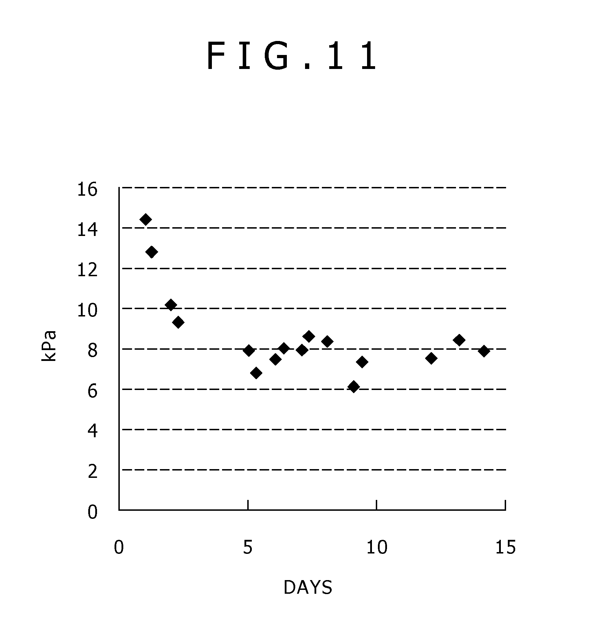

   FIG. 11 a graph showing variations in pressure before correction made each day, for a syringe with a preset pressure of 10 kPa in Experimental Example 2.

### Description of Reference Symbols

- 101: Prefilled syringe (drug container)
- 102: Outer tube
- 103: Plunger
- 104: Cap
- 105: Spring member
- 107: Lock part
- 108: Flange
- 109, 201, 209: Gasket
- 110: Tip opening
- 111: Rear end opening
- 112: Syringe side end part
- 113: Central part
- 114: Intermediate part
- 115: Curved part
- 116: Presser part
- 117: Terminal end part
- 118: Rugged part
- 119: Pressing part
- 120: Hole
- 200, 220: Cartridge
- 202: Glass vial
- 203: Aluminum cap
- 204: Rubber septum
- 205, 210: Fixing cap
- 206: Rib
- 207, 208: Spring member
- 211: Tip part
- 212: Rear end part
- 213, 214: Flange joint
- 501: Measuring syringe
- 502: Pressure gauge
- 503: Pressure regulation port
- 504: Plunger fixing pin
- 505: Luer connector

### Best Mode for Carrying Out the Invention

A characteristic feature of the present invention resides in that in the case where a container in which a liquid drug is enclosed and which is provided at least in a part thereof with a resin member coming into contact with the liquid drug is used, the liquid drug in the drug container is maintained in a pressurized state until immediately before use thereof, by a pressurizing member which is removable from the drug container and operable to maintain the liquid drug in the drug container in a pressurized state, so that the liquid drug in the drug container can be used in a substantially bubble-free state.

Incidentally, the liquid drug in the present invention is an aqueous liquid obtained through dissolution, dispersion or the like of a drug in water.

In general, resin materials have a property of permitting gases such as oxygen, nitrogen, etc. to permeate therethrough by dissolution and diffusion, and the present invention has been made paying attention to this property.

Now, the present invention will be specifically described using a prefilled syringe 101, which is one example of embodiment of the invention, as shown in FIGS. 1 to 5.

The prefilled syringe 101 includes a cylindrical outer tube 102 made of a resin, a resin gasket 109 contained in the outer tube 102 and having a sealing function of being slidable in a liquid-tight manner in relation to an inner surface of the outer tube 102, a plunger 103 extending from a rear end opening 111 of the outer tube 102 and attached to or attachable to the gasket 109, a cap 104 for sealing a tip opening 110 of the outer tube 102, and a spring member 105 formed of an elastic material for pressurizing a liquid drug. Further, a flange 108 extending in the direction of the circumference of the outer tube 102 is provided at the rear end opening 111 of the outer tube 102.

The prefilled syringe 101 can eject the liquid drug filled therein, by removing the cap 104 and pushing the plunger 103 toward the side of the tip opening 110. In addition, where a rubber disk which can be pierced by a needle into the prefilled syringe 101 in a liquid-tight manner is provided at the tip opening 110 of the outer tube 102, the liquid drug can be ejected by use of a double ended needle.

The liquid drug inside the prefilled syringe 101 is isolated from the exterior by the outer tube 102, the gasket 109 and the cap 104.

The spring member 105 (pressurizing member) is made of a substantially angular U-shaped elastic member, and includes a flat central part 113, intermediate parts 114 bent at both end portions of the central part 113 and gently curved, curved parts 115 each curved from the intermediate part 114 toward the inside of the angular U shape in a substantially arcuate shape, presser parts 116 each continuous with the curved part 115 and having an outer surface facing the central part 113, and terminal end parts 117 each curved from the presser part 116 so as to extend away from the central part 113. The presser parts 116 are each engaged with a rugged part 118 provided at that surface of the flange 108 which is on the side of the tip of the syringe, and an inside surface of the central part 113 abuts on a pressing part 119 of the plunger 103 to form a lock part 107. The distance between the central part 113 and the presser part 116 in the syringe axial direction determined by the intermediate part 114 and the curved part 115 is set to be shorter than the distance between the presser part 119 and that surface of the flange 108 which is on the syringe tip side when a predetermined amount of a liquid drug is contained in the outer tube 102. With this setting, the spring member 105 is mounted to the pressing part 119 and the flange 108 while deforming the intermediate parts 114 and the curved parts 115, whereby the spring member 105 is made to generate a force for pushing in the plunger 103 toward the syringe tip side. As a result, an internal pressure is generated in the liquid drug contained in the prefilled syringe 101. The pressing force (pressure) can be varied by regulating the shape and/or material of the spring member 105 and the position of its engagement with the flange 108. In addition, with each of the curved part 115 provided with a hole 120 in its portion making contact with the outer tube 102, it is ensured that a side surface of the outer tube 102 enters the holes 120 in the curved parts 115, and the intermediate parts 114 are shifted toward the inside of the angular U shape, so that the area occupied by the prefilled syringe 101 in the condition where the spring member 105 is mounted can be reduced.

FIGS. 4 and 5 show a part of the spring member 105, in the condition where it is mounted to the prefilled syringe 101 and in the condition where it is dismounted from the prefilled syringe 101, respectively. In the condition where the spring member is dismounted from the prefilled syringe 101 (FIG. 5), no load is exerted thereon. In this condition, the axial distance B between the presser part 116 and the lock part 107 of the spring member 105 is set to be shorter than the axial distance A between the presser part 116 and the lock part 107 in the condition where the spring member 105 is mounted to the prefilled syringe 101 (FIG. 4).

As a result, in the condition where the spring member 105 is mounted to the prefilled syringe 101, a force for returning the axial distance between the presser part 116 and the lock part 107 to B is generated in the spring member 105. By this force, the spring member 105 exerts a compressive force on the liquid drug inside the syringe outer tube 102 from the gasket 109 through the plunger 103.

Incidentally, in using the prefilled syringe 101, the spring member 105 in this shape is rotated in the circumferential direction of the outer tube 102, with the moving direction of the plunger 103 as an axis of rotation, whereby the presser parts 116 are disengaged from the flange 108 and the spring member 105 can be easily removed.

Examples of the material which can be used for the spring member 105 include elastically deformable plastics such as polyethylene, polypropylene, polyvinyl chloride, polyurethane, etc. Also, metallic materials such as stainless steel may also be used insofar as they are elastically deformable.

In addition, the spring member used in the present invention is basically required only to generate a force for constantly pressing the plunger 102. Therefore, the shape and structure of the spring member are not limited to those in this embodiment, and various configurations may be contemplated, for example, one in which the flange 108 and the pressing part 119 are tied together by a rubber. In that case, examples of the material which can be used for the spring member include rubbers such as silicone rubber, urethane rubber, fluoro-rubber, etc. and various elastomers based on olefin, nylon or urethane.

FIGS. 6 to 9 show a container for connection to an ejection device, which is one example of a second embodiment of the present invention.

FIGS. 6 and 7 illustrate an example of application of the present invention to a cartridge to be connected to an insulin injection device (described in U.S. Patent Nos. 5,295,976 and 5,271,527, or the like) such as an insulin pen.

A cartridge 200 includes a glass-made cylindrical glass vial 202 opening at a tip part 211 and a rear end part 212, a resin gasket 201 contained in the glass vial 202 and having both a spring part 207 and a sealing function of being slidable in a liquid-tight manner in relation to an inner surface of the glass vial 202, a fixing cap 205 detachably attached to the gasket 201 and disengageably engaged with the rear end part 212, a rubber septum 204 for sealing the opening at the tip part 211, and an aluminum cap 203 for maintaining a liquid-tight sealed state of the rubber septum 204 relative to the glass vial 202. Further, the glass vial 202 has a rib 206 projecting from the rear end part 212 in the direction of the circumference.

The gasket 201 is provided on the rear end part 212 side thereof with the spring part 207 which is slidably engaged with the fixing cap and has rubber elasticity. The spring part 207 is designed in such a manner that, when the gasket 201 is so mounted that the cartridge 200 is filled up with a liquid drug so as to leave no gas region in the cartridge 200, the rear end of the spring part 207 extends from the opening at the rear end part 212 of the glass vial 202, as shown in FIG. 6.

The fixing cap 205 has a flange joint 213 capable of fitting onto the rib 206, and a part to be slidably engaged with the spring part 207. With the flange joint 213 pushed so as to fit onto the rib 206 as shown in FIG. 7, the spring part 207 is deformed in compression, generating a force for pushing the gasket 201 toward the tip part 211. As a result, a pressure can always be exerted on the liquid drug inside the cartridge 200. In other words, in this embodiment, a combination of the spring part 207 and the fixing cap 205 constitutes a pressurizing member.

The cartridge 200 is connected to an injection device by a method in which before mounting to a device such as a pump, the fixing cap 205 is removed and, thereafter, a central portion of the aluminum cap 203 for fixing the rubber septum 204 is peeled off so as to permit a needle to pierce the rubber septum 204. Therefore, the pressure inside the cartridge 200 has already been released at the time of mounting to the device, so that the liquid drug would not jet out of the cartridge 200 at the time of mounting.

Incidentally, FIGS. 8 and 9 illustrate a partial modification of the above-described second embodiment. A cartridge 220 in this embodiment differs from the above-described cartridge 200 in configuration of the pressurizing member for exerting a compressive force on the liquid drug. A spring part 208 in this modification including a spring which is disposed between a gasket 209 and a fixing cap 210, is formed from an elastic material and is removable from the gasket 209. In other words, the pressurizing member in this modification is constituted of a combination of the spring member 208 separated from the gasket 209 with the fixing cap 209. With the configuration in this modification, also, an effect equivalent to that of the gasket 201 in the above-described embodiment can be attained. In the gasket 201, the gasket (seal) and the spring part (a part of the pressurizing member) are integral with each other; on the other hand, in this modification, the gasket 209 is separate from the spring part (a part of the pressurizing member). The former permits a reduction in the number of component parts, and is easy to assemble.

As a material of the drug container to be used in the present invention, a suitable material can be selected according to the kind of the liquid drug with which the container is filled. Desirably, a material which does not affect the compositions of the liquid drug and additives during storage is used.

For instance, in the case where a liposoluble vitamin compound or phenol ordinarily added to insulin as a combined preservative and stabilizer or the like is contained in the drug to be enclosed in the drug container according to the present invention, use of polypropylene, which is generally used as material of containers, may lead to adsorption of the drug components onto the inside wall of the container or diffusion of the drug components into the outside air. Thus, there arises a need to change the material of the container according to the kind of the liquid agent with which the container is filled.

As has been described above, it is basically difficult for the resin components such as plastics or rubbers to be set to be perfectly impermeable to oxygen or nitrogen molecules. Therefore, the present invention can offer the desired effect if a resin component is used in a part of the container, and it is possible to cope with any change in the kind of material for the main body of the drug container according to the above-mentioned requirements.

For example, even in the case where the container body is made of a perfectly gas-impermeable glass, as shown in FIG. 6, the gasket 201 and the rubber septum 204 are made of resin, so that gas exchange can be made between the inside of the container and the outside air. Consequently, a liquid drug can be stored in the container in such a manner as to prevent generation of bubbles, as above-mentioned.

Thus, it suffices that the blank material of the container pertaining to the present invention is provided at least in a part thereof with a material permitting diffusion therethrough of atmospheric gases such as oxygen and nitrogen.

Examples of the blank material which can be used for forming a hard part of the drug container according to the present invention include polyethylene, polypropylene, cyclic polyolefin, polyethylene terephthalate, polybutylene terephthalate, polymethylpentene-1, mixtures thereof, and admixtures thereof with a softening agent such as SEBS.

Besides, preferable examples of material for the gasket (seal) part and the septum include silicone rubber, flexible polyolefin admixed with a styrene elastomer such as SEBS, etc., butyl rubber, and so on, to which carbon black or ceramic may be added.

Besides, where the above-mentioned blank material is used in a part of the drug container so as to be interposed between the liquid drug and the outside air, it is also possible to use glasses, ceramics, and metals such as stainless steel.

In the present invention, the pressure to be loaded on the liquid drug differs depending on various conditions, such as the blank material and structure of the container, the amount of dissolved gas in the liquid drug and temperature at the time of filling the container with the liquid drug, and how much time it may take for the bubbles generated after filling to disappear.

In the cases of prefilled syringes having a syringe outer tube made of polypropylene, which is used for those prefilled syringes and ordinary syringes which are commercially available at the time of filing of the present patent application, in general, a pressure of not less than 25 kPa is preferable for causing bubbles to disappear during storage at room temperature.

In the case where the dissolved gas upon filling is removed or where heating to 50°C or above can be performed after filling, however, a pressure of about 10 kPa is sufficient for preventing generation of bubbles, or for removing generated bubbles and storing the liquid drug in a bubble-free state.

In addition, where the drug container in the present invention has an autoclave sterilization step, the solubility of gas is lowered at high temperatures and, simultaneously, the partial pressures of atmospheric air components such as oxygen gas and nitrogen gas can be lowered. Consequently, the amount of dissolved gas in the liquid drug can be reduced.

Therefore, with a pressure preliminarily loaded on the liquid drug at the time of autoclave sterilization, it is possible, even by application of a low pressure, to remove gas present in the liquid drug, to put the liquid drug into a state of containing less dissolved gas upon cooling, and to store the liquid drug without generation of bubbles.

In the case of a protein preparation such as insulin, if a liquid drug before aseptic filling is decompressed through a gas exchange membrane to remove dissolved gas therefrom and thereafter filling is conducted and the liquid drug is stored at a temperature of below 10°C, even application of a pressure of about 5 kPa is sufficient for storing the liquid drug without generation of bubbles.

However, even in the case of a protein preparation, the drug is normally used at room temperature. Therefore, even in the case of a liquid drug stored at a low temperature, it is preferable to store the liquid drug under an applied pressure of not less than 10 kPa.

Now, the effect of the present invention will be described below referring to experimental examples. Incidentally, the liquid drug used in the experimental examples was selected to show a condition where bubbles are liable to be generated. Specifically, ultrafiltered water left to stand for at least one day with occasional stirring in a 4°C cold storage in the condition where a gas-liquid interface was present (hereinafter referred to as "cold-stored water") was used as a liquid drug model.

### (Experimental Example 1)

FIG. 10 illustrates a measuring apparatus used in the experimental examples.

A commercial vacant 10 ml (this expression shows the state of being commercialized as a product for a 10 ml capacity, here and hereinafter) polypropylene syringe 501 (Terumo Syringe, a product by Terumo Corporation) was filled with water having been stored with cooling at 4°C, in the manner of not leaving any gas region inside the syringe 501. Further, a pressure gauge 502 (PG-35, a product by Nidec Copal Corporation) was connected to the syringe 501 through a Luer connector 505.

A gasket of this commercial syringe is formed of a flexible polyolefin elastomer admixed with a styrene elastomer.

The plunger was fixed by a plunger fixing pin 504 so that the position of the plunger relative to an outer tube is kept unchanged, whereby the pressure exerted on the water was prevented from being lowered due to movement of the gasket. Auxiliary parts other than the syringe, such as the pressure gauge 502 and a pressure regulation port 503, were formed from brass or stainless steel so as to prevent penetration of gas through the auxiliary parts, and the inside of the auxiliary parts was filled up with water.

An initial pressure was set by pouring the water into the syringe through the pressure regulation port 503, the syringe 501 was left to stand for 1 hr in a 50°C oven, and thereafter the presence or absence of bubbles inside the syringe 501 was visually checked. The results are shown in Table 1.

**(Table 1) Generation of bubbles during storage at 50°C in 10 ml polypropylene syringe**

| Initial pressure (kPa) | After 1 hr at 50°C | |
|---|---|---|
| | Pressure (kPa) | Confirmation of bubbles |
| 0.1 | 20.1 | present |
| 10.0 | 23 | present |
| 15.2 | 28.6 | present |
| 20.0 | 36.2 | present |
| 25.6 | 26.8 | present |

From the results of Experimental Example 1, it was confirmed that the storage at 50°C raised the pressure inside each syringe to 20 kPa or above, resulting in generation of bubbles in all the syringes. This shows that the pressure rise is caused by a phenomenon in which the solubility of dissolved gas is lowered due to the temperature rise and bubbles are thereby generated.

### (Experimental Example 2)

The same syringes as above were treated and measurement was conducted in the same measuring conditions as in Experimental Example 1, except that the internal pressure was regulated to a preset value after the lapse of 1 hr from transfer into the 50°C oven and, thereafter, the water-filled syringes were stored for a long time while correcting the pressure to the preset value every day. The results are shown in Table 2.

**(Table 2) Change of bubbles with time at 50°C**

| Preset pressure | At pressure confirmation and correction after storage at 50°C | | | Confirmation of disappearance of bubbles |
|---|---|---|---|---|
| (kPa) | Max-P (kPa) | Min-P (kPa) | Av(kPa)±SD | |
| 0 | 9.6 | 1.3 | 4.0±2.3 | Not disappeared in 2 weeks |
| 10 | 14.4 | 2.5 | 8.4±2.6 | Not disappeared in 2 weeks |
| 20 | 20.5 | 10.0 | 16.0±2.6 | Disappeared in 8 days |
| 27 | 23.3 | 22.7 | 23.0±0.4 | Disappeared in 2 days |
| 30 | 26.5 | 14.8 | 20.8±5.7 | Disappeared in 2 days |

From the results of Experimental Example 2, it was confirmed that bubbles inside the syringes are caused to disappear when a pressure of not less than 20 kPa is continuously exerted on the aqueous liquid in the syringes. It was also confirmed that the rate of disappearance of bubbles increases as the pressure is raised, and that it is possible by raising the pressure to cause disappearance of bubbles in about two days.

Incidentally, even when the preset pressure was 20 kPa, for example, the pressure would be lowered with time. In the measuring apparatus shown in FIG. 10 used in the experimental examples, generation or disappearance of extremely little bubbles could even be detected as pressure changes with good sensitivity, since the syringes had a substantially constant internal volume.

Accordingly, this lowering in pressure can be considered to be due to release of gas component out of the syringes.

Where no pressure is exerted, on the other hand, the pressure inside the container is somewhat raised due to generation of bubbles. It can be understood, however, that it is difficult for the bubbles to disappear under this pressure rise.

Specifically, the foregoing shows that, at a syringe internal pressure of about 10 kPa, the diffusion of gas molecules toward the inside of the syringe and the diffusion of the gas molecules toward the outside of the syringe are in such a relation that the gas diffusion toward the outside of the syringe is not predominant, and, therefore, bubbles would not disappear. It is also shown that when the pressure is further raised to 20 kPa, on the other hand, the release of gas from the inside of the syringe into the atmosphere, or the gas diffusion toward the outside of the syringe, becomes predominant. Thus, the following can be said. If transpiration of water inside the syringe into the atmosphere does not occur, that is, if the species exchanged through the resin wall surfaces are only such gas components as oxygen and nitrogen molecules, a condition where the pressure inside the syringe becomes substantially constant with time is a condition where the amounts of gas components diffused toward the inside of the syringe and those toward the outside of the syringe are balanced.

In the case where a pressure of 10 kPa is exerted on water, the pressure at the time of correction varies as shown in FIG. 11. Immediately after the start of the test, generation of bubbles was present, and the pressure at the time of correction was above 10 kPa. On the other hand, after several days, a constant pressure value was attained which was around 8 kPa and approximate to the preset pressure. This shows that a pressure of about 8 to 10 kPa is the pressure at which the amount of gas diffused toward the outside of the syringe and the amount of gas diffused toward the inside of the syringe are balanced.

However, even in the case of polypropylene, which has a low water vapor permeability, the rate of permeation of water vapor is not 0 (zero). In other words, the volume of water in a syringe made of polypropylene decreases with time, and the pressure is also liable to be lowered according to the decrease in the volume of water inside the syringe. Accordingly, the above-mentioned pressure at which the rates of diffusion of gas components other than water, such as dissolved oxygen and nitrogen, toward the outside of the syringe and the rates of diffusion of the gas components toward the inside of the syringe are balanced is a value obtained by subtracting the lowering in pressure due to transpiration of water vapor. Thus, the balancing pressure can be estimated to be higher than the pressure relevant to the boundary between disappearance of bubbles and non-disappearance of bubbles.

In considering the condition inside the syringe, it is to be noted that the inside of bubbles is filled with saturated water vapor; therefore, to obtain the pressure of the gas components such as oxygen and nitrogen molecules in the bubbles, the pressure of saturated water vapor must be subtracted from the internal pressure of the syringe. In order that the coming-in and coming-out of oxygen and nitrogen molecules through the resin serving as a partition between the inside and the outside of the syringe are balanced, it is considered necessary for the respective gas partial pressures in the inside of the syringe to be equal to the corresponding gas partial pressures in the outside of the syringe. This means that when the water inside the syringe is pressurized by a value equal to the saturated water vapor pressure, the partial pressures of the oxygen and nitrogen gases in the inside of the syringe are balanced with those in the outside of the syringe. In fact, saturated water vapor pressure is 12.2 kPa at 50°C, which satisfies the results shown in FIG. 2. Specifically, in the case of storing and distributing the drug containers of the present invention by maintaining them at a temperature of not higher than room temperature, prevention of generation of bubbles and removal of generated bubbles can be achieved even with a lower container internal pressure, since the saturated water vapor pressure at room temperature of 20°C is 2.3 kPa. However, during normal distribution, the drug containers may be momentarily exposed to elevated temperatures. Taking this into account, it is preferable to adopt a condition in which bubbles would not be generated and bubbles, if generated, can be removed at a temperature of around 50°C. Thus, it is preferable to maintain a syringe internal pressure of not less than 10 kPa.

The time of two days required for disappearance of bubbles, at preset pressures of 27 kPa and 30 kPa, is sufficiently short as compared with the time necessary for getting lot control data for guarantee, distribution thereof, or the like after the manufacture of the products. This shows that there is no problem on a practical-use basis.

In addition, this experimental example was started from a condition in which bubbles were highly liable to be generated. Therefore, it is clear that if a contrivance of filling the liquid drug into the drug container after degassing the liquid drug or in a bubble-free state is additionally adopted, the syringe used in this experimental example ensures that generation of bubbles is prevented, also during distribution period, even by pressurizing to a pressure of about 10 kPa.

### (Experimental Example 3)

Disappearance of bubbles during storage under pressurizing to 25 kPa was confirmed by the same method as in Experimental Example 2, except that the storage temperature was changed to room temperature and commercial syringes of various capacities (5 ml: Vitaject, a product by Terumo Corporation, 10 ml: Terumo Syringe, a product by Terumo Corporation, 20 ml: KCl Mediject, a product by Terumo Corporation) were used.

The materials constituting the components of each syringe were polypropylene for the outer tube, and a styrene elastomer-containing olefin elastomer for the gasket.

The results are shown in Table 3.

**(Table 3) Disappearance of bubbles in polypropylene syringes of various capacities**

| Preset pressure (kPa) | At pressure confirmation and correction after storage at room temperature | | | Confirmation of disappearance of bubbles |
|---|---|---|---|---|
| | Max-P (kPa) | Min-P (kPa) | Av(kPa)±SD | |
| 5 ml Syringe | 17.1 | 12.2 | 14.9±2.5 | Disappeared in 3 days |
| 10 ml Syringe | 10.4 | 5.0 | 8.1±2.5 | Disappeared in 6 days |
| 20 ml Syringe | 21.0 | 17.7 | 19.2±1.7 | Disappeared in 5 days |

From the results of Experimental Example 3, bubbles were confirmed in all syringes, upon the process of a temperature rise from a 4°C cold storage state to room temperature. It was thereby confirmed that in syringes different in capacity and shape, bubbles formed from gases dissolved during cold storage can be removed within one week in the condition of storage at room temperature.

Incidentally, the 10 ml syringe was the same as that used in Experimental Example 2. In this case, with the temperature lowered from 50°C to room temperature (about 20°C), the time necessary for disappearance of bubbles was prolonged to about three times the original value.

In other words, while a higher temperature is more effective in removing bubbles, it was found possible by the present invention to remove bubbles sufficiently effectively even during storage at room temperature.

### (Experimental Example 4)

A 5 ml syringe used for a commercial prefilled syringe (Vitaject, a product by Terumo Corporation), using cyclic polyolefin as a material of the outer tube, was prepared. Using the same measuring apparatus as used in Experimental Example 1, the syringe was filled with cold-stored water (having been stored at 4°C), the pressure was set at 25 kPa, and the water-filled syringe was left to stand in a 50°C oven for one hr. Incidentally, the gasket was formed of butyl rubber.

As a result, the pressure rose to 88.7 kPa, but bubbles were not observed.

From the results of Experimental Example 4, it was found that also in the case of a syringe formed using cyclic polyolefin for the outer tube, generation of bubbles can be prevented if the pressure loaded on the liquid drug is about 90 kPa at maximum.

### (Experimental Example 5)

Using the same prefilled syringes and measuring apparatus as those used in Experimental Example 4, the regulation ports of the syringes were kept open for one hr after placing the syringes in a 50°C oven, to lower the pressure to zero, with the result of generation of bubbles. Thereafter, the pressure was regulated to 25 kPa for one syringe and 50 kPa for the other syringe. For each of the syringes, the pressure was confirmed and regulated once a day, and variation of bubbles with time was observed.

From the results of Experimental Example 5, it was confirmed that at the pressure of 25 kPa, the bubbles would not disappear in two weeks but that at the pressure of 50 kPa, the bubbles disappeared within one week. Besides, after the disappearance of the bubbles, the bubble-free syringe was left to stand at 50°C and 50 kPa for two days, then the pressure was returned to the atmospheric pressure, and the syringe was left to stand in that condition for one hr. Consequently, bubbles were not generated.

From the foregoing, it was found that, even with differences in materials for syringe and gasket and the like, fine bubbles generated at the time of filling respective syringes with a liquid drug can be removed, though there are differences in the pressure and time required.

## Claims

1. A drug container, in which a liquid drug is enclosed, provided at least in a part thereof with a resin coming into contact with the liquid drug, wherein a pressurizing member removable from the drug container and operable to maintain the liquid drug in a pressurized state is provided, and the liquid drug is filled in a substantially bubble-free state.

2. The drug container according to claim 1, wherein the drug container comprises an outer tube having a tip and a rear end, and a seal slidable on an inner surface of the outer tube in an axial direction of the outer tube, the tip being sealed with a sealing member, at least one of the seal and the outer tube is made of the resin member, and the liquid drug is placed in the outer tube between the sealing member and the seal.

3. The drug container according to claim 2, wherein a plunger extending from the rear end side of the outer tube and having a tip part and rear end part is provided, the tip part being connected with the seal, the rear end part having a pressing part; and the pressuring member is an elastic member which is removably mounted by a flange's surface on the side of the tip of the outer tube and the pressing part and which gives a compressive force between the flange and the pressing part.

4. The drug container according to claim 2, wherein the pressurizing member is comprised of an elastic member which has a fixation part for fixation to the outer tube and which is detachably attached to the seal.

5. The drug container according to claim 2, wherein the pressurizing member has a fixation part for fixation to the outer tube, and maintains the liquid drug in a pressurized state through pressing of an elastic member of the seal provided with the elastic member.

6. The drug container according to any of claims 1 to 5, wherein an internal pressure relevant to the pressurized state in which the liquid drug is maintained is 10 to 90 kPa.
